# NEUE EUROPÄISCHE PATENTSCHRIFT

(11) **EP 3 578 917 B2**
(45) Veröffentlichungstag und Bekanntmachung des Hinweises auf die Entscheidung über den Einspruch: **07.01.2026**
(45) Hinweis auf die Patenterteilung: 09.11.2022
(21) Anmeldenummer: 19178444.6
(22) Anmeldetag: 05.06.2019
(51) Int. Cl.: G01B 15/04, G01B 11/25, G01N 33/02, B26D 7/06

(54) **VORRICHTUNG UND VERFAHREN ZUM SCANNEN VON LEBENSMITTELRIEGELN MIT VERFAHRBARER SCANEINHEIT**
DEVICE AND METHOD FOR SCANNING FOOD BARS WITH MOVABLE SCANNING UNIT
DISPOSITIF ET PROCÉDÉ DE BALAYAGE DES ALIMENTS EN BÂTON POURVU D'UNITÉ DE BALAYAGE MOBILE

(30) Priorität: 07.06.2018 DE 102018113618
(43) Veröffentlichungstag der Anmeldung: 11.12.2019
(73) Patentinhaber: Weber Food Technology SE & Co. KG, 35236 Breidenbach (DE)
(72) Erfinder: von KEUDELL, Leopold, 88348 Bad Saulgau (DE); WEBER, Tobias, 35216 Biedenkopf-Wallau (DE)
(74) Vertreter: Grünecker Patent- und Rechtsanwälte PartG mbB

(56) Entgegenhaltungen:
- EP-A1- 1 741 525
- EP-A1- 2 711 701
- EP-A1- 3 081 090
- WO-A2-2010/112239
- DE-A1- 102012 018 754
- DE-A1- 102016 114 477
- DE-A1- 19 642 159
- DE-A1- 3 246 568
- DE-A1- 3 246 568
- US-A- 4 937 451
- US-A- 5 754 617

## Beschreibung

Die vorliegende Erfindung betrifft eine Scanvorrichtung zum Scannen von Lebensmittelriegeln für ein Lebensmittelverarbeitungssystem, mit einer Fördereinrichtung zum Fördern eines Lebensmittelriegels in einer Längsrichtung, und einer Scaneinheit, die ausgelegt ist, die äußere und/oder innere Gestalt wenigstens eines Lebensmittelriegels zu erfassen. Die vorliegende Erfindung bezieht sich weiterhin auf ein Verfahren zum Scannen von Lebensmittelriegeln, bei dem ein Lebensmittelriegel in einer Längsrichtung mittels einer Fördereinrichtung gefördert wird, und der Lebensmittelriegel mittels einer Scaneinheit gescannt wird.

Im Stand der Technik ist aus der DE 10 2012 018 754 A1 und der EP 2 711 701 A1 eine Lebensmittelbearbeitungsvorrichtung bekannt, in der Lebensmittelprodukte auf parallelen, separat antreibbaren Förderspuren nacheinander durch einen Scanbereich eines Scanners gefördert werden können.

Weiterhin ist es aus der WO 2007/049305 A1 bekannt, ein Stück Fleisch mittels eines Röntgenapparats und eine Dickenbestimmungsvorrichtung zu untersuchen, und dessen Eigenschaften zu quantifizieren.

Aus der WO 2010/112239 A2 ist eine Lebensmittelaufschneidevorrichtung mit Durchstrahlscanner bekannt, wobei sowohl das zu scannende Lebensmittelprodukt als auch der Durchstrahlscanner beweglich sein können.

Aus der US 5,754,617 ist ein Röntgensystem zur Durchleuchtung von Frachtcontainern bekannt, wobei eine Röntgeneinheit von einem ersten zu einem zweiten Ende eines Containers bewegt werden kann, um diesen zu durchleuchten.

Aus der US 4,937,451 ist eine Röntgenapparatur bekannt, bei welcher in einem Endlosförderband mittels Umlenkrollen eine Bandlücke gebildet wird, sodass ein Objekt zwischen Röntgenquelle und Detektor hindurchbefördert werden kann, ohne dass das Förderband ebenfalls durchleuchtet wird.

Aus der DE 32 46 568 A1 ist ein Vorratsbehälter für flockenartiges Material bekannt, wobei das Material aus einem Abwurfzyklon durch eine Bandlücke zwischen zwei gegenläufigen Förderbändern nach unten in einen Behälter fällt und bei ansteigendem Füllstand an die jeweilige Außenseite gefördert wird.

Gattungsgemäße Scanvorrichtungen weisen zumeist ein Gehäuse auf, insbesondere um Röntgenstrahlung der Scaneinheit abzuschirmen, oder um zu verhindern, dass eine lichtbasierte Messung durch Licht aus der Umgebung in ihrer Messgenauigkeit beeinträchtigt wird.

Die im Stand der Technik bekannten Scaneinrichtungen weisen oft eine erhebliche Länge und damit ein großes Gehäuse auf, was zu hohem Konstruktionsaufwand und einer Verlängerung der Linienlänge der Lebensmittelverarbeitungslinie führt.

Es ist die Aufgabe der vorliegenden Erfindung eine Scanvorrichtung und ein Verfahren zum Scannen von Lebensmittelriegeln bereitzustellen, die kompakte Vorrichtungsabmessungen ermöglichen und einen effizienten Betrieb.

Die Erfindung stellt eine Scanvorrichtung gemäß dem unabhängigen Anspruch 1 zum Scannen von Lebensmittelriegeln für ein Lebensmittelverarbeitungssystem bereit, mit einer Fördereinrichtung zum Fördern eines Lebensmittelriegels in einer Längsrichtung, und einer Scaneinheit, die ausgelegt ist, die äußere und/oder innere Gestalt wenigstens eines Lebensmittelriegels zu erfassen, wobei erfindungsgemäß die Scaneinheit wenigstens entlang der Längsrichtung verfahrbar ist. Durch die in Längsrichtung verfahrbare Scaneinheit kann der Lebensmittelriegel während des Scannens stationär gehalten werden oder in Ergänzung der Bewegung der Scaneinheit in Längsrichtung vornehmlich entgegen dieser Bewegung verfahren werden. Damit wird der während des Scanvorgangs für den Lebensmittelriegel erforderliche Raum reduziert. Weiterhin kann die Scaneffizienz gesteigert werden, wenn durch Kombination der Bewegung von Lebensmittelriegel und Scaneinheit in Gegenrichtung eine Beschleunigung des Scannens ermöglicht wird. Der Scanvorgang ermittelt durch ein bildgebendes Verfahren Informationen über die äußere und/oder innere Gestalt des Lebensmittelriegels. Die Lebensmittelriegel sind insbesondere in Förderrichtung der Scanvorrichtung ausgerichtet, die somit der Längsrichtung entspricht.

Bei dem Lebensmittelverarbeitungssystem handelt es sich vorzugsweise um ein System, das stromabwärts der Scanvorrichtung eine Schneidmaschine aufweist, mit der die gescannten Lebensmittelriegel in Scheiben aufgeschnitten können, wobei das Scanergebnis bei der Steuerung des Aufschneidens berücksichtigt werden kann, um die Scheibendicken anzupassen und/oder eine Klassifizierung oder Gruppierung der Scheiben vorzunehmen. Weiterhin kann eine Verpackungsmaschine stromabwärts der Scanvorrichtung vorgesehen sein. Eine zentrale Steuerung und/oder ein Kommunikationsmittel, wie z.B. ein Bussystem, kann die vorgenannten Komponenten des Lebensmittelverarbeitungssystems verbinden, um einen Datenaustausch zwischen den Komponenten zu ermöglichen.

Bei den Lebensmittelriegeln handelt es sich insbesondere um länglich geformte Lebensmittelprodukte mit konstantem oder in Längsrichtung variierendem Querschnitt, die homogen oder inhomogen aufgebaut sein können. Insbesondere handelt es sich bei den Lebensmittelriegeln um Käsestangen, Wurststangen, längliche Schinkenlaibe und andere länglich geformte Lebensmittelprodukte.

Insbesondere können mehrere länglich geformte Lebensmittelriegel in einer Förderspur hintereinander aufgereiht und zusammen gescannt werden, beispielweise mehrere kurze Schinken- oder Käseriegel in einer Anordnung hintereinander. Damit kann eine für längere Lebensmittelriegel ausgelegte Scanvorrichtung auch für kürzere Lebensmittelriegel effizient verwendet werden.

In einer bevorzugten Ausführungsform umfasst die Scanvorrichtung ein Schutzgehäuse, mit einem Innenraum, der ausgelegt ist, den wenigstens einen Lebensmittelriegel während des Scanvorgangs aufzunehmen, sodass dieser darin angeordnet werden kann, wobei die Länge des Innenraums des Schutzgehäuses in Längsrichtung kürzer ist als die zweifache Länge eines Lebensmittelriegels. Damit kann der Lebensmittelriegel während des gesamten Scanvorgangs im Schutzgehäuse angeordnet sein. Im Stand der Technik, ohne die in Längsrichtung verfahrbare Scaneinheit, ist hingegen ein Gehäuse notwendig, das wenigstens die zweifache Länge des Lebensmittelriegels aufweist, da der Lebensmittelriegel während des Scanvorgangs komplett durch die Scanebene gefahren werden muss, sodass er davor vollständig vor der Scanebene angeordnet ist, und danach vollständig hinter der Scanebene. Die Länge eines Lebensmittelriegels ist dessen maximale Erstreckung in Längsrichtung, mit oder ohne Berücksichtigung von dessen durchmesserreduzierten, insbesondere abgerundeten Endstücken. Die Lebensmittelriegel weisen vornehmlich eine Länge zwischen 15 cm und 180 cm, insbesondere zwischen 20 cm und 100 cm, auf. Besonders Naturprodukte weisen eine unregelmäßige Form und/oder Struktur auf und werden vorteilhafterweise zuführseitig entsprechend vermessen.

Die Scanvorrichtung ist so eingerichtet, dass die Scaneinheit unterbrechungsfrei entlang der gesamten Länge des Lebensmittelriegels während des Scannens verfahren wird.

Vorteilhafterweise weist das Schutzgehäuse einen öffenbaren und schließbaren Eingang sowie einen öffenbaren und schließbaren Ausgang auf. Insbesondere handelt es sich dabei um Öffnungen im Schutzgehäuse, die durch schwenkbare oder verschiebbare Tore geöffnet bzw. geschlossen werden können. Die Tore können vorteilhafterweise durch von einer zentralen Steuerung ansteuerbare Aktoren geöffnet bzw. geschlossen werden. Je nach Scantechnik kann somit verhindert werden, dass Röntgenstrahlung nach außen austritt bzw. dass das Scannen im Inneren des Schutzgehäuses aufgrund von äußeren Einflüssen, wie zum Beispiel Umgebungslicht, negativ beeinflusst wird.

Die Lebensmittelriegel sind vorteilhafterweise während des Scanvorgangs vollständig in dem Schutzgehäuse aufgenommen.

In einer Ausführungsform weist die Scaneinheit eine Strahlungsquelle, insbesondere eine Röntgenvorrichtung, auf, die ausgelegt ist, den Lebensmittelriegel zu durchstrahlen. Damit können Informationen über den inneren Aufbau des Lebensmittelriegels erhalten werden, insbesondere über die Dichteverteilung darin. Dafür sind eine Strahlungsquelle und eine Detektoreinheit auf gegenüberliegenden Seiten des Lebensmittelriegels angeordnet.

Insbesondere erfolgt das Durchstrahlen entlang einer Scanebene oder Röntgenebene, deren Normale im Wesentlichen in Richtung der Längsrichtung orientiert ist. Je dichter und höher der Lebensmittelriegel entlang einer Strahlenbahn ist, umso mehr Strahlung wird absorbiert. Die Detektoreinheit bestimmt die Intensität der Strahlung, wodurch durch Vergleich mit der Energie der Strahlungsquelle oder mit Referenzmessungen, Rückschlüsse auf die Dichte und/oder Höhe des Lebensmittelriegels gewonnen werden.

Die Detektoreinheit kann insbesondere ähnlich einer Zeilenkamera aufgebaut sein, die jeweils den Verlauf der Absorption in Breitenrichtung des Lebensmittelriegels erfassen kann. Dabei werden während des Verfahrens der Scaneinheit scheibchenweise Messungen in Längsrichtung des Lebensmittelriegels durchgeführt, sodass sich am Ende eine Dichtekarte in Längs- und Breitenrichtung des gesamten Lebensmittelriegels oder wenigstens für dessen zum Aufschneiden vorgesehenen Bereich errechnet werden kann. Alternativ kann in der Scaneinheit eine Computertomografie-Röntgenvorrichtung vorgesehen sein, die pro Messung jeweils Dichtekarten in Hoch- und Breitenrichtung erstellt, sodass durch Verfahren der Scaneinheit in Längsrichtung eine 3D-Dichtekarte in Hoch-, Breiten- und Längsrichtung errechnet werden kann.

Alternativ oder zusätzlich zur der Röntgenvorrichtung kann ein Geometriescanner vorgesehen sein. Der Geometriescanner kann durch ein optisches Messverfahren die äußere Kontur des Lebensmittelriegels wenigsten bereichsweise bestimmen. Der Geometriescanner kann als Teil der Scaneinheit vorgesehen sein, und als Teil dieser in Längsrichtung verfahrbar sein. Der Geometriescanner kann der Röntgenvorrichtung vorgeschaltet oder sogar direkt in diese integriert sein.

Alternativ kann der Geometriescanner aber auch stationär vorgesehen sein und beispielsweise die Lebensmittelriegel beim Einfahren in die Scanvorrichtung, insbesondere in deren Schutzgehäuse, scannen. Der Geometriescanner umfasst insbesondere einen Laserstrahler, der eine Linie auf den Lebensmittelriegel projiziert. Die aufprojizierte Linie wird von einer Kamera erfasst, die in einem anderen Winkel angeordnet ist als der Laserstrahler. Aus dem Verlauf der Linie kann die äußere Kontur des Lebensmittelriegels berechnen werden. Wird der Lebensmittelriegel durch den Geometriescanner gefahren bzw. der Geometriescanner entlang des Lebensmittelriegels gefahren, so kann die gesamte Außenkontur des Lebensmittelriegels bestimmt werden. Folglich ist das Volumen des Lebensmittelriegels bekannt.

Die mit dem Geometriescanner bestimmten Eigenschaften des Lebensmittelriegels können zur Steuerung der Röntgenvorrichtung dienen, insbesondere um deren Leistungen in Abhängigkeit vom Volumen zu regeln, mit dem Ziel die Strahlenbelastung so gering wie möglich zu halten.

Der Geometriescanner kann an einer beliebigen Position in oder am Schutzgehäuse vorgesehen sein. Bei der Erfassung der Geometrie oberhalb einer (geschlossenen) Auflage kann der Auflagebereich des Lebensmittelriegels per Extrapolation mitbestimmt werden.

Gewöhnliche Röntgenverfahren, wie sie derzeit vornehmlich bei Röntgenvorrichtungen für Lebensmittelriegel eingesetzt werden, können nicht zwischen äußeren geometrischen Veränderungen und inneren geometrischen Veränderungen unterscheiden. Ebenso kann eine Dichteveränderung nicht immer sicher von einer Veränderung in der Zusammensetzung des Lebensmittelriegels unterschieden werden.

Durch die zusätzliche Erfassung des Volumens des Lebensmittelriegels mittels eines Geometriescanners wird eine größere Differenzierung ermöglicht, da entlang einer Röntgenstrahlrichtung der Eintrittspunkt und Austrittspunkt des Röntgenstrahls im Lebensmittelriegel ermittelt werden können.

Das mit dem Geometriescanner erfasste Volumen und der mit der Röntgenvorrichtung erfasste Datensatz können gemeinsam ausgewertet werden, um z.B. den Einfluss der Außengeometrie des Lebensmittelriegels auf die Absorption der Röntgenstrahlen zu kompensieren.

Durch das geometrisch erfasste Volumen kann bestimmt werden, ob äußere oder innere Veränderungen in den Lebensmittelriegeln vorliegen. Ein Beispiel für eine innere Veränderung ist ein Gärnest in einem Backprodukt, wie beispielsweise Brot. Ein Beispiel für eine äußere Formänderung ist beispielsweise ein Gärungsriss in einem Backprodukt, beispielsweise in einer Brotoberfläche.

Die durch die Kombination von Geometriescanner und Röntgenvorrichtung erfassten Scandaten enthalten detailliertere Informationen über den Aufbau des Lebensmittelriegels, und ermöglichen eine verbesserte Weiterverarbeitung des Lebensmittelriegels. Auf Basis der Scandaten kann direkter Einfluss auf den Aufschneidebetrieb bzw. Schneidplan einer Aufschneidemaschine genommen werden. Zum Beispiel kann ein Fehlerbereich im Lebensmittelriegel als Ausschuss eingeplant werden, und später ausgeschleust werden. Weiterhin kann ein Lebensmittelriegel aus dem Prozess aussortiert bzw. speziell klassifiziert werden.

In einer Ausführungsform weist die Fördereinrichtung ein umlaufendes Fördermittel auf, wobei zwischen dessen oberem Auflagebereich und unterem Rücklaufbereich eine Detektoreinheit der Röntgenvorrichtung vorgesehen ist. Alternativ kann in dieser Position aber auch eine Strahlungsquelle vorgesehen sein, und die Detektoreinheit über dem Lebensmittelriegel angeordnet sein.

Das umlaufende Fördermittel kann durch ein Unterstützungsmittel unterstützt werden, damit der Lebensmittelriegel in einer definierten Position, vorteilhafterweise in einer horizontalen Ebene, gehalten wird. Das Unterstützungsmittel ist insbesondere direkt unter dem fördernden bzw. oberen Trum des umlaufenden Fördermittels vorgesehen.

Insbesondere kann ein Unterstützungsmittel in Form einer flachen oder konkaven plattenförmigen Unterlage vorgesehen werden, auf der das Fördermittel läuft, sodass das Fördermittel und der Lebensmittelriegel definiert unterstützt und in Position gehalten werden. Das Unterstützungsmittel weist vorzugsweise eine gleichmäßige Stärke auf, und erstreckt sich durchgehend über die Lebensmittelriegellänge bzw. den Auflagebereich für den Lebensmittelriegel.

Weiterhin ist auch möglich, als Unterstützungsmittel lediglich bereichsweise unterstützende Elemente unter dem Fördermittel vorzusehen, so dass lediglich eine schmale Zone unterstützt wird. Beispielsweise können Längsstreben als Unterstützungsmittel vorgesehen werden. Insbesondere können zwei Längsstäbe vorteilhaft im seitlichen Randbereich des Fördermittels angeordnet werden. Dies ermöglicht, die Störeinflüsse auf den durch die Scaneinheit durchgeführten Scanvorgang gering zu halten.

In einer Ausführungsform ist es möglich, dass das Unterstützungsmittel gemeinsam mit der Scaneinheit verfahrbar ist. Insbesondere kann dabei eine Ausnehmung im Unterstützungsmittel in der Scanebene vorgesehen sein. Dadurch können negative Effekte, insbesondere Abschirmungseffekte, beim Scanvorgang vermieden werden.

Das Unterstützungsmittel besteht vorzugsweise aus einem Material, das wenig Röntgenstrahlung absorbiert.

Wenn die Lage und insbesondere das Material des Unterstützungsmittels bekannt ist oder ein Referenzbild ohne Lebensmittelriegel erstellt wurde, so kann aus dem mit der Scaneinheit ermittelten Datensatz der Störeinfluss des Unterstützungsmittels herausgerechnet werden, sodass ein genaueres Bild nur vom Lebensmittelriegel erzeugt wird.

Insbesondere kann das Referenzbild in regelmäßigen Abständen erstellt werden, um beispielsweise eine Verschmutzung der Fördermittel durch Anhaftung von Lebensmittelbestandteilen zu kompensieren, oder andere Förderbänder, Riemen oder Ketten zu kompensieren, wenn diese zum Einsatz kommen. Insbesondere kann das Referenzbild nach Inbetriebnahme, nach Reinigung oder nach Verarbeitung einer Lebensmittelriegelcharge neu erstellt werden. Bei dem umlaufenden Fördermittel handelt es sich vorteilhafterweise um wenigstens ein Förderband, um wenigstens eine Förderkette, oder wenigstens einen Förderriemen.

Gemäß der Erfindung umfasst die Fördereinrichtung ein umlaufendes Fördermittel, wobei eine durch Umlenkrollen begrenzte und in Längsrichtung verfahrbare Bandlücke im oberen Auflagebereich der Fördereinrichtung vorgesehen ist. Insbesondere ist die Scaneinheit auf die Bandlücke ausgerichtet und gemeinsam mit dieser verfahrbar. Somit wird ein Störeinfluss des Fördermittels auf den Scanvorgang vermieden. Der Lebensmittelriegel erstreckt sich während des Scanvorgangs über der Bandlücke und diese kann entlang dessen Längserstreckung verfahren werden, vorteilhafterweise während der Lebensmittelriegel im Wesentlichen stationär gehalten wird. Insbesondere umfasst die Fördereinrichtung nur ein umlaufendes Fördermittel. In anderen Ausführungsformen können auch mehrere separate Fördermittel vorgesehen werden, wobei die Bandlücke insbesondere zwischen ein-und ausgangsseitig korrespondierenden, umlaufendem Fördermitteln, insbesondere Förderbändern, vorgesehen ist.

In einer Ausführungsform weist die Fördereinrichtung zwei Rückzugsbänder auf, deren in Längsrichtung verfahrbare Umlenkrollen die Ränder der Bandlücke definieren. Die Ausgestaltung als zwei separate Rückzugsbänder ermöglicht insbesondere, dass unterschiedliche Fördergeschwindigkeiten sowie eine flexible Verstellung der Breite der Bandlücke erfolgen können. Allerdings ist es möglich, auch bei nur einem umlaufenden Fördermittel eine Verstellung der Breite der Bandlücke in Längsrichtung zu ermöglichen, indem ein Längenausgleichselement in dem Fördermittel vorgesehen wird, beispielsweise eine verstellbare Umlenkrolle.

Insbesondere kann die Breite der Bandlücke reduziert werden, wenn der Lebensmittelriegel vor dem Scannen in die Scanvorrichtung eingefahren wird. Dies ermöglicht ein sicheres und schnelles Fördern des Lebensmittelriegels. Während des Scanvorgangs wird die Bandlücke dann auf die notwendige Breite gestellt, um ein störungsfreies Scannen des Lebensmittelriegels zu ermöglichen.

Die Umlenkrollen können mechanisch oder steuerungstechnisch in Längsrichtung gekoppelt sein, um eine gleichbleibende Länge der Bandlücke zu gewährleisten. Insbesondere umfasst diese Kopplung auch die Röntgeneinrichtung bzw. Scaneinheit. Vorzugsweise kann die mechanische Kopplung durch ein mechanisches Verbindungselement, beispielsweise eine Verbindungsstange, gebildet werden. Die an die Bandlücke grenzenden Umlenkrollen können jeweils mit dem Verbindungselement verbunden oder in diesem gelagert sein. In manchen Ausführungsformen kann die Scaneinheit wenigstens zeitweise, d.h. insbesondere während des Scanvorgangs, mit der Bandlücke mechanisch oder steuerungstechnisch in Längsrichtung gekoppelt sein, sodass die Scaneinheit nur gemeinsam mit der Bandlücke verfahrbar ist.

In einer bevorzugten Ausführungsform weist die Fördereinrichtung mehrere parallele Förderspuren auf. Damit kann der Durchsatz durch die Scanvorrichtung erhöht werden. Insbesondere ist es möglich, dass ein Lebensmittelriegel in einer der parallelen Förderspuren gescannt wird oder mehrere Lebensmittelriegel in einer Untergruppe der parallelen Förderspuren gescannt werden, während die Lebensmittelriegel in anderen parallelen Förderspuren in die Scanposition gefahren werden oder in der Scanposition warten.

Bei einer mehrspurigen Scanvorrichtung kann sich der Scanvorgang über zumindest zwei Spuren erstrecken. Über eine Bilddatenauswertung erfolgt dann eine Zuordnung der gemessenen Daten auf die jeweilige Spur und den dort gescannten Lebensmittelriegel.

In einer mehrspurigen Scanvorrichtung kann ein breites Fördermittel für mehrere Lebensmittelriegel mit einer durchgehenden Bandlücke vorgesehen sein. Andernfalls kann auch wenigstens ein separates Fördermittel pro Spur vorgesehen sein.

In einer Ausführungsform ist die Scaneinheit zwischen den Förderspuren in Querrichtung verfahrbar. Damit wird ermöglicht, dass mit einer Scaneinheit Lebensmittelriegel in mehreren Förderspuren sequenziell gescannt werden können.

In einer Ausführungsform kann eine in Längsrichtung verfahrbare Scaneinheit pro Förderspur oder pro einer Untergruppe von Förderspuren vorgesehen sein. Damit können zeitgleich die Lebensmittelriegel in jeder Förderspur oder in jeder Untergruppe der Förderspuren gescannt werden. In einer Ausführungsform ist eine gemeinsame Scaneinheit für alle Spuren vorgesehen. Damit können alle Lebensmittelriegel in allen Spuren gleichzeitig gescannt werden.

In einer Ausführungsform sind die parallelen Förderspuren unabhängig voneinander betreibbar. Folglich können die Lebensmittelriegel in den einzelnen Spuren nicht nur gemeinsam, sondern alternativ sequenziell einzeln oder sequenziell in Gruppen durch die Scanvorrichtung bewegt werden. Dies hat den Vorteil, dass der Scanvorgang flexibler gestaltet werden kann, insbesondere können Lebensmittelriegel auf verschiedenen Spuren sequenziell und/oder unabhängig zugefördert, gescannt und abgefördert werden. Folglich kann die Effizienz der Scanvorrichtung gesteigert werden, und bei einer Reduzierung der Anzahl der gleichzeitig gescannten Lebensmittelriegel kann die notwendige Scanleistung der Scaneinheit, insbesondere die Stärke der emittierten Röntgenstrahlung, reduziert werden.

Vorzugsweise ist eine Steuerung, die insbesondere einen Mikrocontroller umfasst, vorgesehen, wobei mittels der Steuerung ein Antrieb des Fördermittels, ein Antrieb für die Scaneinheit, und optional ein Antrieb für die Positionierung der Bandlücke ansteuerbar sind. Mit dem Antrieb des Fördermittels kann insbesondere die Fördergeschwindigkeit des Fördermittels vorgegeben werden. Mit dem Antrieb der Scaneinheit kann insbesondere die Verfahrbewegung der Scaneinheit entlang der Längsrichtung gesteuert werden. Mit dem Antrieb für die Positionierung der Bandlücke können vorzugsweise die an die Bandlücke angrenzenden bzw. die die Bandlücke definierenden Umlenkrollen gemeinsam oder unabhängig voneinander in Längsrichtung verfahren werden. Weiterhin können gegebenenfalls Ausgleichselemente verfahren werden, die sicherstellen, dass trotz des Verfahrens der Umlenkrollen die vorgegebene Spannung des Förderbands erhalten bleibt. Das Verfahren der Ausgleichselemente kann durch den Antrieb für die Positionierung der Bandlücke bewirkt werden, oder durch einen separaten Antrieb.

Die Erfindung stellt weiterhin ein Verfahren gemäß dem unabhängigen Anspruch 9 zum Scannen von Lebensmittelriegeln bereit, das das Fördern eines Lebensmittelriegels in einer Längsrichtung mittels einer Fördereinrichtung und das Scannen des Lebensmittelriegels mittels einer Scaneinheit umfasst, wobei erfindungsgemäß die Scaneinheit entlang des Lebensmittelriegels in Längsrichtung während des Scannens verfahren wird.

Vorteilhafterweise wird die Scaneinheit, insbesondere unterbrechungsfrei, entlang der gesamten Länge des Lebensmittelriegels während des Scannens verfahren.

Das Fördern des Lebensmittelriegels kann während des Scannens unterbrochen werden. Folglich kann der Lebensmittelriegel stationär angeordnet verbleiben, während die Scaneinheit entlang des Lebensmittelriegels verfahren wird. Dies ermöglicht einen kompakten Aufbau der Fördereinrichtung und Scaneinheit.

Das Fördern des Lebensmittelriegels kann während des gesamten Scannen unterbrochen werden, sodass der Lebensmittelriegel während des gesamten Scanvorgangs stationär ist, während die Scaneinheit verfahren wird. Alternativ kann der Lebensmittelriegel wenigstens zeitweise oder aber auch die gesamte Zeit während des Scannens bewegt werden, insbesondere in Förder- bzw. Längsrichtung und relativ zu der sich bewegenden Scaneinheit. Dabei wird der Lebensmittelriegel aber nur auf einer begrenzten Strecke bewegt, die insbesondere durch die Größe des Schutzgehäuses vorgegeben wird.

In einer Ausführungsform kann das Fördern des Lebensmittelriegels das Einfahren des Lebensmittelriegels in ein Schutzgehäuse umfassen, wobei die Scaneinheit nur innerhalb des Schutzgehäuses verfahren wird. Folglich ist es möglich, den Scanvorgang von äußeren Einflüssen abzuschirmen, und/oder zu verhindern, dass Röntgenstrahlung aus dem Schutzgehäuse austritt.

Insbesondere bei optischen Scanverfahren kann das Verfahren auch ohne geschlossenes Schutzgehäuse betrieben werden. Dann kann das Scannen auch während des Ein- und/oder Ausfahren des Lebensmittelriegels durchgeführt werden.

In einer Ausführungsform werden die Lebensmittelriegel in mehreren parallellen Spuren gefördert, wobei die Scaneinheit zur Durchführung von Scanvorgängen in verschiedenen Spuren in Querrichtung zwischen den Spuren verfahren wird. Damit kann mit einer Scaneinheit, die lediglich für das Scannen in der Breite einer Spur oder einer Untergruppe von Spuren ausgelegt ist, sequentielles Scannen in mehreren parallelen Spuren oder Untergruppen von Spuren ermöglicht werden.

Vorteilhafterweise wird die Scaneinheit bei aufeinanderfolgenden Scanvorgängen jeweils in entgegengesetzte Richtungen verfahren. Insbesondere wird die Scaneinheit abwechselnd in die Förderrichtung und in die Gegenförderrichtung verfahren. Dazwischen wird die Scaneinheit vorteilhafterweise in Querrichtung zwischen den Spuren verfahren. Damit können mehrere Scanvorgänge schnell aufeinanderfolgen, da kein Rückfahren der Scaneinheit in Ausgangsposition notwendig ist.

In einer Ausführungsform wird der Lebensmittelriegel beim Scannen durchstrahlt, und das Ergebnis des Scanvorgangs mit Referenzdaten korrigiert, die bei einem Scanvorgang ohne Lebensmittelriegel ermittelt wurden. Insbesondere weisen diese Referenzdaten Informationen bezüglich der in der Scanebene angeordneten Komponenten der Fördereinrichtung auf, die zusammen mit dem Lebensmittelriegel durchstrahlt werden. Insbesondere kann die Fördereinrichtung Komponenten in Form von Fördermitteln, wie beispielsweise Bänder, Riemen oder Ketten, oder Unterstützungsmitteln, wie Unterstützungsplatten oder-stäbe, aufweisen, die gemeinsam mit dem Lebensmittelriegel durchstrahlt werden. Im Rahmen der Referenzdatenerfassung wird die Auswirkung der durchstrahlten Komponenten auf die Scandaten erfasst, sodass in nachfolgenden Scanvorgängen eine Korrektur der Scandaten des Lebensmittelriegels erfolgen kann.

Gemäß der Erfindung wird die Scaneinheit während des Scannens gemeinsam mit einer Bandlücke der Fördereinrichtung verfahren, sodass die Scanebene während des Scannens stets innerhalb der Bandlücke liegt. Somit kann beim Einsatz einer Röntgenvorrichtung vermieden werden, dass der Lebensmittelriegel zusammen mit der Fördereinrichtung durchstrahlt wird. Vielmehr wird nur der Lebensmittelriegel durchstrahlt, sodass die Fördereinrichtung das Messergebnis nicht beeinflusst. Bei einem entsprechenden Einsatz eines Geometriescanners kann der Lebensmittelriegels von allen Seiten erfasst werden.

Während des Scannens und der entsprechenden Verschiebung der Bandlücke, wird der Lebensmittelriegel bevorzugt stationär gehalten, nämlich durch einen entsprechend abgestimmten Antrieb der Fördereinrichtung.

Außerhalb des Scanvorgangs ist es aber auch möglich, dass der Lebensmittelriegel mit einer unterschiedlichen Geschwindigkeit bewegt wird als die Scaneinheit bzw. die Bandlücke. Die Geschwindigkeit des Lebensmittelriegels wird durch den Antrieb der Fördereinrichtung bestimmt, die Geschwindigkeit der Scaneinheit durch die Geschwindigkeit des Bandlücken-Positionierungs-Antriebs bzw. durch den Antrieb der Scaneinheit entlang der Längsrichtung. Damit kann die Scaneinheit und Bandlücke in eine Ausgangsposition zurückgefahren werden oder in eine beliebige, lebensmittelriegelabhängig vorgebbare Ausgangslage, während der Lebensmittelriegel der Scanvorrichtung zu- oder abgefördert wird.

Ein beispielhafter Ablauf des erfindungsgemäßen Verfahrens kann wie folgt zusammengefasst werden:
Zunächst wird ein Lebensmittelriegel in das Schutzgehäuse mittels Antrieb eines entsprechenden Fördermittels eingefahren. Die Scaneinheit ist an einem in Längsrichtung und Querrichtung verfahrbaren Portal angeordnet. Dabei fährt das Portal die Scaneinheit in einen stromabwärtigen Endbereich einer ersten Förderspur. Das Portal bewegt die Scaneinheit dann entgegen der Förderrichtung über den gesamten Lebensmittelriegel komplett hinweg, sodass der Lebensmittelriegel vollständig gescannt wird. Die Scaneinheit steht nun am stromaufwärtigen Endbereich und wird mittels des Portals quer zur parallelen Spur verfahren. Das Portal fährt die Scaneinheit dann in Förderrichtung über den auf der zweiten Spur angeordneten Lebensmittelriegel, sodass auch dieser Lebensmittelriegel vollständig gescannt wird. Dann werden die Lebensmittelriegel gemeinsam aus dem Schutzgehäuse ausgefahren.

In einer alternativen Ausführungsform kann die Scaneinheit so ausgebildet sein, dass parallel mehrere Förderspuren gleichzeitig gescannt werden können. Bei dieser Alternative können mehrere, insbesondere spurindividuelle Scaneinheiten vorgesehen sein, welche entweder mit einem Portal gemeinsam oder mit separaten Halterungen spurindividuell in der Längsrichtung bewegt werden können.

Die Bewegungsrichtung der Scaneinheit während des Scannens entlang der Längsrichtung ist beliebig, d.h. es kann ein Scannen insbesondere in stromaufwärtiger als auch in stromabwärtiger Richtung erfolgen. Vorteilhafterweise wird die Scaneinheit bei aufeinanderfolgenden Scanvorgängen jeweils in gegenüberliegende Richtungen bewegt, sodass Rückstellvorgänge der Scaneinheit vermieden werden können.

Die vorliegende Erfindung wird nun anhand von in den beiliegenden Figuren dargestellten Ausführungsformen beispielhaft beschrieben.
Figur 1 zeigt eine schematische Seitenansicht einer ersten Ausführungsform einer erfindungsgemäßen Scanvorrichtung.
Figur 2 zeigt eine schematische Seitenansicht einer zweiten Ausführungsform einer erfindungsgemäßen Scanvorrichtung.
Figur 3 zeigt eine schematische Seitenansicht einer dritten Ausführungsform einer erfindungsgemäßen Scanvorrichtung.
Figuren 4a bis 4d zeigen eine Draufsicht auf eine vierte Ausführungsform einer erfindungsgemäßen Scanvorrichtung und illustrieren den Ablauf einer Ausführungsform eines erfindungsgemäßen Verfahrens.

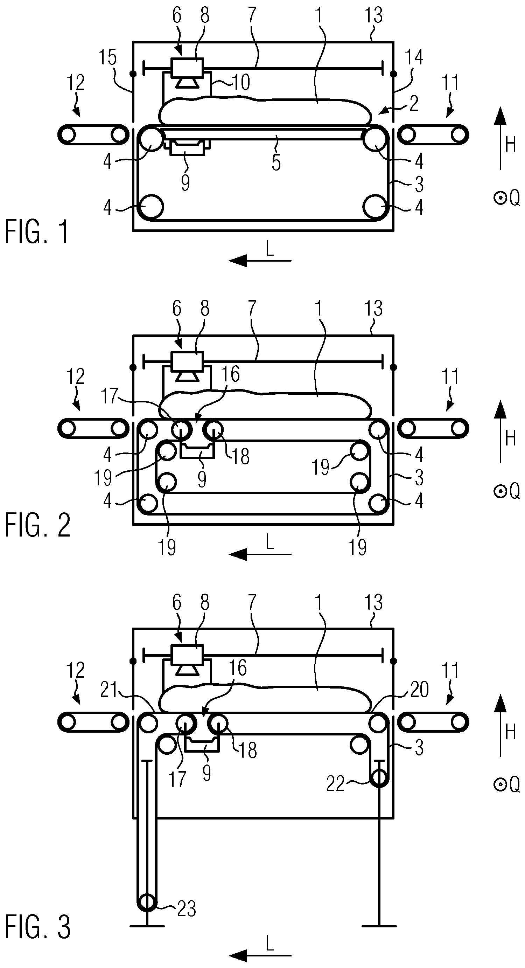

In Fig. 1 wird eine Scanvorrichtung und ein darin angeordneter Lebensmittelriegel 1 in Seitenansicht gezeigt. Die Scanvorrichtung weist eine Fördereinrichtung 2 auf, die ein Fördermittel 3 in Form eines um mehrere Umlenkrollen 4 umlaufenden Förderbands umfasst. Die Fördereinrichtung 2 weist weiterhin ein Unterstützungsmittel 5, insbesondere in Form einer Unterlage, die durch eine Platte gebildet wird, auf. Alternativ können als Unterstützungsmittel 5 auch ein, zwei oder mehrere Unterstützungsstäbe vorgesehen sein. Das Unterstützungsmittel 5 ist direkt unter dem Fördermittel in dessen Förderbereich angeordnet, und stellt sicher, dass das Fördermittel in einer vorbestimmten Position gehalten wird, auch wenn darauf ein Lebensmittelriegel 1 mit einem höheren Gewicht angeordnet ist. Weiterhin umfasst die Fördereinrichtung 2 einen Antrieb für das Fördermittel 3, der den Umlauf des Fördermittels 3 um die Umlenkrollen 4 steuert, und somit den Lebensmittelriegel in Förderrichtung bzw. Längsrichtung L fördert.

Weiterhin ist eine Scaneinheit 6 vorgesehen, die wenigstens entlang der Längsrichtung L verfahrbar ist. Die Längsrichtung erstreckt sich vorteilhafterweise in horizontaler Richtung. Die Scanebene hat eine Normale in Längsrichtung, erstreckt sich also in Hochrichtung H und Querrichtung Q.

Die Scaneinheit 6 ist entlang einer Aufhängung oder Schiene 7 in Längsrichtung L verschiebbar. Die Bewegung der Scaneinheit 6 wird durch einen Antrieb vorgegeben, bei dem es sich insbesondere um einen Linear-Antrieb, beispielsweise einen Magnetlinearantrieb, aber auch um einen Kettenantrieb, Hydraulik- oder Pneumatik-Antrieb oder Ähnliches handeln kann. Die Scaneinheit 6 umfasst eine Strahlungsquelle 8, die insbesondere eine Röntgenröhre umfasst, und eine Detektoreinheit 9. Bei der Detektoreinheit 9 handelt es sich insbesondere um eine sich in Querrichtung Q erstreckende Zeilenkamera, die digital die Intensität der Röntgenstrahlung in der Scanebene erfassen kann.

Die Scaneinheit 6 ist so installiert, dass eine Beweglichkeit über den gesamten Auflagebereich des Lebensmittelriegels 1 gegeben ist. Insbesondere liegt eine axiale Verfahrbarkeit bezüglich der Längsrichtung L des Lebensmittelriegels 1 vor, damit ein Röntgenscannen im Durchlauf des Lebensmittelriegels erfolgen kann.

Alternativ kann die Scaneinheit als Geometriescanner ausgeführt sein. Dann handelt es sich bei der Strahlungsquelle um einen Lichtstrahler, insbesondere einen Laserstrahler, und bei der Detektoreinheit um eine optische Kamera. Allerdings sind die Strahlungsquelle und die Detektoreinheit dann nicht wie dargestellt gegenüberliegend zueinander angeordnet, sondern in einem Winkel kleiner als 180°, da dann jeweils die Reflektion vom Lebensmittelriegel und nicht dessen Durchstrahlung gemessen wird. In einer Ausführungsform kann die Scaneinheit sowohl eine Röntgenvorrichtung als auch einen Geometriescanner umfassen.

Die Strahlungsquelle 8 und die Detektoreinheit 9 sind vorteilhafterweise über eine mechanische Kopplung 10, in Form eines Rahmens oder einer Koppelstange, verbunden, sodass sie gemeinsam verschoben werden können. In anderen Ausführungsformen ist es allerdings auch möglich, dass die Steuerungseinheit und Detektoreinheit in separaten Führungen vorgesehen und individuell beweglich sind, und für den Scanvorgang lediglich synchron bewegt werden, d.h. steuerungstechnisch gekoppelt werden.

Die Strahlungsquelle 8 befindet sich vorzugsweise oberhalb der Auflageebene und die Detektoreinheit 9 befindet sich unterhalb des oberen Auflagetrums des Fördermittels 3. Die Strahlungsquelle 8 und die Detektoreinheit 9 bilden eine integrale Röntgenvorrichtung mit fest zueinander zugeordneten Komponenten.

In Fig. 1 ist die Scaneinheit 6 in einer Position nahe ihrer stromabwärtigsten Position dargestellt, und wird ausgehend davon während des Scanvorgangs entlang der Längsrichtung L des Lebensmittelriegels 1 bewegt, nämlich entgegen der Längsrichtung L. In einem darauffolgenden Scanvorgang kann die Scaneinheit 6 dann in Längsrichtung L des Lebensmittelriegels 1 bewegt werden.

Durch die Bewegung der Scaneinheit 6 entlang des Lebensmittelriegels 1, kann durch scheibchenweises bzw. diskretes oder kontinuierliches Scannen in der entlang des Lebensmittelriegels verfahrenen Scanebene der gesamte Lebensmittelriegel 1 messtechnisch erfasst werden.

Aus der den Lebensmittelriegel 1 durchdringenden Strahlung kann eine Dichtekarte des gesamten Lebensmittelriegels 1 ermittelt werden. Dafür wird die Scaneinheit 6 vom stromabwärtigen Ende zum stromaufwärtigen Ende des Lebensmittelriegels 1 verfahren oder alternativ von dessen stromaufwärtigen Ende zu dessen stromabwärtigen Ende.

Insbesondere ist eine Zuführfördereinrichtung 11 vorgesehen, mit der der Lebensmittelriegel 1 der Scanvorrichtung zugeführt werden kann. Weiterhin ist eine Abführfördereinrichtung 12 vorgesehen, mit der der Lebensmittelriegel 1 von der Scanvorrichtung ausgehend weiter gefördert werden kann. Bei der Zuführfördereinrichtung 11 und der Abführfördereinrichtung 12 kann es sich insbesondere um Komponenten von vor- bzw. nachgeordneten Stationen handeln. Beispielsweise kann die Abführfördereinrichtung 12 Teil einer Beladefördereinrichtung einer Lebensmittelaufschneidemaschine sein. Die mit der Scanvorrichtung ermittelten Daten können bei der Steuerung der Lebensmittelaufschneidemaschine berücksichtigt werden.

Die Scanvorrichtung umfasst ein Schutzgehäuse 13, das ein Eingangstor 14 und ein Ausgangstor 15 aufweist. Bei den Toren 14, 15 handelt es sich insbesondere um schwenkbare oder verschiebbare Tore, die mit einem Antrieb versehen sind, um geöffnet bzw. geschlossen zu werden. Das Eingangstor 14 kann geöffnet werden, um einen Lebensmittelriegel in die Scanvorrichtung einzufahren. Während des Scanvorgangs werden die Tore geschlossen gehalten. Nach Abschluss des Scanvorgangs wird das Ausgangstor 15 geöffnet, um den Lebensmittelriegel aus der Scanvorrichtung auszufahren.

Insbesondere wird mindestens in einer Spur gescannt, es kann aber auch parallel oder sequenziell in mehreren parallelen Spuren gescannt werden.

Eine Ausführungsform eines erfindungsgemäßen Verfahrens mit der Scanvorrichtung gemäß Fig. 1 stellt sich wie folgt dar. Ausgehend von einer Zuführfördereinrichtung 11 wird der Lebensmittelriegel 1 mittels die Fördereinrichtung 2 durch das Tor 14 in das Schutzgehäuse 13 der Scanvorrichtung hineingefahren und das Tor geschlossen.

Dann wir die Scaneinheit 6 in Längsrichtung L verfahren, um den gesamten Lebensmittelriegel zu scannen, während die Tore 14, 15 geschlossen sind. Der Lebensmittelriegel wird während des Scannens nicht bewegt. Die Scandaten werden dann auf Basis eines Referenzbildes oder durch Korrekturwerte hinsichtlich der Fördereinrichtung korrigiert.

Dann wird der Lebensmittelriegel aus dem Schutzgehäuse 16 mittels der Fördereinrichtung 2 durch das Tor 15 auf das Abführfördermittel 12 ausgefahren.

Fig. 2 zeigt eine weitere Ausführungsform einer erfindungsgemäßen Scanvorrichtung. In dieser Ausführungsform weist die Fördereinrichtung 2 eine Bandlücke 16 auf, die durch zwei voneinander beabstandete Umlenkrollen 17, 18 der Fördereinrichtung 2 definiert wird. Wie in Fig. 2 dargestellt, sind die Umlenkrollen in Längsrichtung L miteinander mechanisch gekoppelt, sodass ein vorbestimmter Abstand zwischen ihnen eingehalten und somit die Breite der Bandlücke 16 vorgegeben wird.

Das Fördermittel 3 läuft nicht nur um die äußeren Umlenkrollen 4, sondern auch um innere Umlenkrollen 19. Folglich ist es möglich, dass eine Bandlücke vorhanden ist, obwohl nur ein integrales umlaufendes Fördermittel 3 zum Einsatz kommt. In der in Fig. 2 gezeigten Ausgestaltung ist die Breite der Bandlücke konstant. Optional kann eine nicht dargestellte, verstellbare Ausgleichsrolle vorgesehen sein, die insbesondere translatorisch verfahren werden kann, um die Breite der Bandlücke einzustellen.

In der Ausführungsform gemäß Fig. 2 ist die Strahlungsquelle 8 der Scaneinheit 6 nicht mit der Detektoreinheit 9 mechanisch gekoppelt, sondern wird nur synchron mit dieser bewegt, d.h. steuerungstechnisch gekoppelt. Alternativ ist aber auch eine mechanische Kopplung möglich.

In einer alternativen Ausführungsform kann eine weitere Bandlücke im unteren Trum der Fördereinrichtung 2 vorgesehen werden. Dies ermöglicht, dass die Fördereinrichtung 2 aus zwei unabhängig voneinander antreibbaren Fördermitteln besteht, und somit die Einfahr- bzw. Ausfahrgeschwindigkeit unterschiedlich gestaltet werden kann, oder anders flexibel auf die Förderung des Lebensmittelriegels eingewirkt werden kann. Die Fördermittel sind dann entweder mechanisch oder steuerungstechnisch miteinander und/oder hinsichtlich der Bandlückenbewegung gekoppelt. Dadurch kann insbesondere sichergestellt werden, dass die relative Anordnung der Bandlücke und der beiden Fördermittel zur Scanebene immer gleich bleibt.

Je nach Position des Lebensmittelriegels und der Bandlücke liegt dieser jeweils mehr oder weniger auf den beiden Fördermitteln oder Fördermittelabschnitten vor und nach der Bandlücke auf. Insbesondere kann die Scanebene durch abgestimmte Bewegung der Bandlücke und des oder der Fördermittel relativ zum Lebensmittelriegel bewegt werden. Bei einem Ein- oder Ausfahren des Lebensmittelriegels wird die Bandlücke durch entsprechendes Antreiben des oder der Fördermittel mit dem Lebensmittelriegel überfahren. Insbesondere kann die Bandlücke dabei stillstehen.

Es ist aber auch möglich, die Bandlücke während des Ein- und Ausfahrens des Produkts zu bewegen, entweder im Rahmen eines Scanvorgangs oder zum Anordnen der Scaneinheit in einer Ausgangsposition. Beim Einfahren des Lebensmittelriegels bringt das Fördermittel den Lebensmittelriegel in Position, d.h. platziert ihn in geeigneter Art und Weise in dem Schutzgehäuse. Dabei kann bereits vor oder während des Einfahrens des Lebensmittelriegels die Scaneinheit und Bandlücke in die Startposition für den nächsten Scanvorgang gefahren werden. Vorteilhafterweise wird die Bandlücke und die Scaneinheit dabei in einem Endbereich an einer der Endseiten des Schutzgehäuses in Längsrichtung positioniert. Sobald die Scaneinheit in die Ausgangsposition für den Scanvorgang verfahren worden ist, wird, falls der Lebensmittelriegel noch nicht richtig positioniert ist, nur noch das oder die Fördermittel angetrieben, um den Lebensmittelriegel vollständig im Gehäuse anzuordnen. Dann werden die Tore des Schutzgehäuses geschlossen und der Scanvorgang durchgeführt. Insbesondere kann zeitgleich ein Lebensmittelriegel in das Schutzgehäuse eingefahren werden, während ein anderer Lebensmittelriegel in der gleichen Spur aus dem Schutzgehäuse ausgefahren wird. Vorteilhafterweise wird nur ein geringer Abstand zwischen den Lebensmittelriegeln in Längsrichtung eingehalten.

Nach dem Scanvorgang steht die Bandlücke im gegenüberliegenden Endbereich zu ihrer vormaligen Startposition. Der nachfolgende Scanvorgang kann dann vorteilhafterweise in der umgekehrten Bewegungsrichtung für die Scaneinheit erfolgen. Alternativ kann die Bandlücke bzw. die Scaneinheit vor oder während des Ausfahrens der Lebensmittelriegel aus der Scanvorrichtung wieder in die Ausgangsposition für den nächsten Durchlauf verfahren werden.

Fig. 3 zeigt eine weitere Ausführungsform einer erfindungsgemäßen Scanvorrichtung. Hier umfasst die Fördereinrichtung 3 ein erstes Fördermittel 20 und ein zweites Fördermittel 21. Das erste Fördermittel 20 ist stromaufwärtig des zweiten Fördermittels 21 angeordnet, und zwischen den Fördermitteln 20, 21 ist eine Bandlücke 16 ausgebildet. Unter der Bandlücke 16 ist eine Detektoreinheit 9 angeordnet. Die Fördermittel 20, 21 weisen an ihrem der Bandlücke 16 zugeordneten Enden jeweils Umlenkrollen 17, 18 auf. Weiterhin weisen die Fördermittel 20, 21 jeweils Ausgleichsrollen 22, 23 auf, die entlang einer Führung, hier insbesondere entlang einer vertikalen linearen Führung, verfahren werden können, um ein Verfahren der Bandlücke 16 in Längsrichtung L zu ermöglichen. Durch die separaten Ausgleichsrollen 22, 23 kann aber auch ohne Weiteres die Breite der Bandlücke 16 in Längsrichtung L angepasst werden.

So kann die Bandlücke nahezu ganz geschlossen werden, während der Lebensmittelriegel 1 in die Scanvorrichtung ein- oder ausgefahren wird. Dafür werden die Umlenkrollen 17, 18 und damit die Bandenden dichter aneinander bewegt. Dies hat insbesondere den Vorteil, dass das Überfahren der Bandlücke bzw. des Bandübergangs lebensmittelriegelschonender ist, wodurch auch die unterhalb der Bandlücke befindliche Detektionseinheit vor Verschmutzung geschützt werden kann.

Während des Scanvorgangs kann die Bandlücke dann auf einen Abstand in Längsrichtung gefahren werden, sodass ein Durchtritt der Strahlen von der Strahlungsquelle 8 zur Detektoreinheit 9 ohne Beeinträchtigung durch das Fördermittel erfolgen kann.

Die Fördermittel 20, 21 sind als zwei separate Rückzugsbänder ausgebildet und vorteilhafterweise steuerungstechnisch, gegebenenfalls aber zusätzlich oder alternativ mechanisch miteinander gekoppelt. Eine mechanische Kopplung ist vorteilhafterweise im Bereich der Bandlücke 16 vorgesehen und koppelt die Bandlücke gegebenenfalls auch mit der Scaneinheit 6. Damit bleibt die relative Anordnung der Bandlücke 16 und der beiden Fördermittel 20, 21 zumindest in Längsrichtung immer gleich.

Die Ausgleichsrollen 22, 23 können unabhängig im umgebenden Schutzgehäuse 13 angeordnet sein oder sich aus diesem heraus erstrecken. Die Lebensmittelriegel liegen während des Scanvorgangs oder des Ein- oder Ausfahrvorgangs jeweils mehr oder weniger auf den beiden Fördermitteln 20, 21 auf.

Eine Positionierung der Lebensmittelriegel bezüglich der Röntgenebene erfolgt durch abgestimmte Fördermittelgeschwindigkeiten der beiden Fördermittel 20, 21, sowie einen abgestimmten Bandrückzug durch Bewegung der Ausgleichsrollen 22, 23. Insbesondere ist eine Steuerung vorgesehen, die die Fördermittelgeschwindigkeit und die Bewegungen der Ausgleichsrollen 22, 23 so vorgibt, dass kein Schlupf am Lebensmittelriegel entsteht.

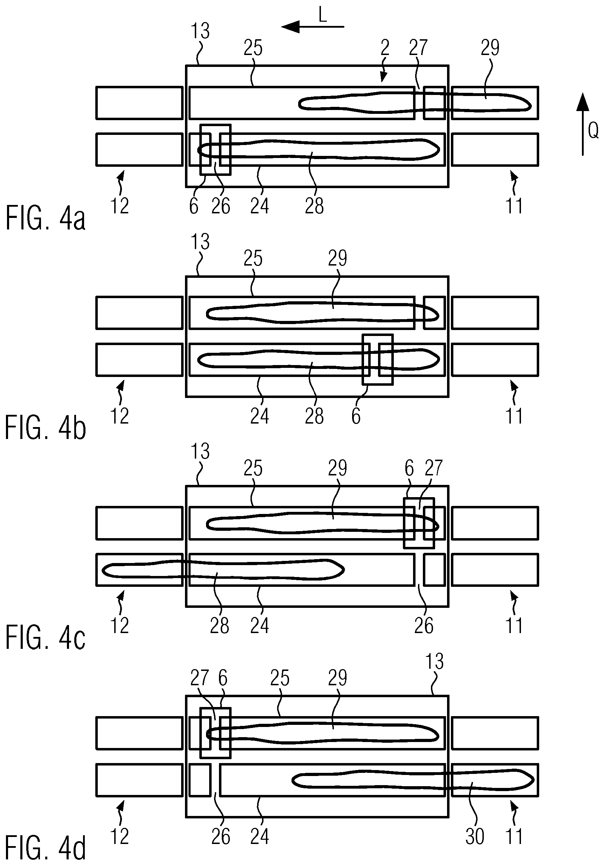

Fig. 4a bis 4d stellen jeweils eine Draufsicht auf eine weitere Ausführungsform einer erfindungsgemäßen Scanvorrichtung zu verschiedenen Betriebszeitpunkten dar.

Die Scanvorrichtung weist eine Fördereinrichtung 2 mit zwei parallelen Fördermitteln 24 und 25 auf, wobei in dem ersten Fördermittel 24 eine erste Bandlücke 26 und in dem zweiten Fördermittel 25 eine zweite Bandlücke 27 vorgesehen sind. Die Gestaltung der Fördermittel hinsichtlich der Bandlücken 26, 27 kann gemäß Fig. 2 oder 3 erfolgen.

Zunächst werden eine Scanvorrichtung 6 und die Bandlücke 26 des ersten Fördermittels 24 in die in Fig. 4a dargestellte Scanausgangsposition bezüglich eines ersten Lebensmittelriegels 28 bewegt. Zeitgleich wird ein zweiter Lebensmittelriegel 29 von der Zuführfördereinrichtung 11 auf dem zweiten Fördermittel 25 in das Schutzgehäuse 13 eingefahren.

Wie in Fig. 4b dargestellt, wird der gesamte erste Lebensmittelriegel 28 durch Verfahren der Scaneinheit 6 entgegen der Längsrichtung L gescannt. Dabei ist der erste Lebensmittelriegel 28 stationär innerhalb des Schutzgehäuses 13 angeordnet. Nachdem der Scanvorgang des ersten Lebensmittelriegels 28 abgeschlossen ist, wird die Scaneinheit in Querrichtung Q zum zweiten Fördermittel 25 bewegt, sodass die Scaneinheit 6 mit dessen Bandlücke 27 ausgerichtet ist, wie in Fig. 4c gezeigt. Dann wird durch Bewegung der Scaneinheit 6 und der Bandlücke 27 in die Längsrichtung L der zweite Lebensmittelriegel 29 entlang seiner gesamten Länge gescannt.

Wie in Fig. 4d im Vergleich zu Fig. 4c gesehen werden kann, wird die Bandlücke 27 des ersten Fördermittels 24 während oder nach dem Scannens des Lebensmittelriegels 29 auf dem zweiten Fördermittel 25 in Längsrichtung L zurück in ihre Ausgangsposition, wie in Fig. 4d und 4a gezeigt, gebracht.

Nach Abschluss des Scannens des zweiten Lebensmittelriegels 29, wird ein dritter Lebensmittelriegel 30 auf das erste Fördermittel 24 eingefahren. Die Scaneinheit 6 wird dann wiederum querverfahren, sodass dann der Lebensmittelriegel 30 auf dem ersten Fördermittel 24 gescannt werden kann.

Insbesondere kann die Scaneinrichtung 6 an einem Portal angebracht sein und kann an diesem Portal längs entlang und quer zwischen den Förderspuren verfahren werden.

Die vorgenannte Ausführungsform mit zwei parallelen Bändern kann auch ohne Bandlücke, beispielsweise mit einem Aufbau gemäß Fig. 1 ausgestaltet werden, wobei dann gegebenenfalls mittels eines Referenzbilds der Produktauflage das gemessene Scanbild korrigiert werden sollte.

Die Erfindung ermöglicht, dass das Schutzgehäuse der Scaneinheit nur etwa so lang wie der längste zu scannende Lebensmittelriegel ist. Dadurch ergibt sich eine verkürzte Linienlänge und eine einfachere Integration der Scanvorrichtung bzw. eine einfachere Aufstellung in einem Produktionsbetrieb. Zudem wird der Bauaufwand reduziert. Weiterhin wird ein Zeitvorteil beim Scanvorgang ermöglicht, da keine Rückstellung notwendig ist, und ein fließendes Ein-und Ausfahren der Lebensmittelriegel ermöglicht wird. Bei einer Kombination von einem Röntgen- und einem Geometriescanner in der Scaneinheit ergibt sich eine modular aufgebaute Scanvorrichtung, die dadurch auch einfach anwendungsabhängig nachrüstbar sein kann.

In alternativen Ausführungsformen kann die Detektoreinheit über dem Lebensmittelriegel angeordnet werden und die Scaneinheit darunter. In anderen Ausführungsformen ist es möglich, die Scaneinheit auf einer Seite in Querrichtung neben dem Lebensmittelriegel anzuordnen und die Detektoreinheit auf der gegenüberliegenden Seite.

## Patentansprüche

1. Scanvorrichtung zum Scannen von Lebensmittelriegeln (1, 28, 29, 30) für ein Lebensmittelverarbeitungssystem, mit
einer Fördereinrichtung (2) zum Fördern wenigstens eines Lebensmittelriegels (1, 28, 29, 30) in einer Längsrichtung (L), und
einer Scaneinheit (6), die ausgelegt ist, die äußere und/oder innere Gestalt des wenigstens einen Lebensmittelriegels (1, 28, 29, 30) zu erfassen, wobei
die Scaneinheit (6) wenigstens entlang der Längsrichtung (L) verfahrbar ist, wobei die Fördereinrichtung (2) ein umlaufendes Fördermittel (3, 20, 21) umfasst, und
**dadurch gekennzeichnet, dass**
eine durch Umlenkrollen (17, 18) begrenzte und in Längsrichtung (L) verfahrbare Bandlücke (16, 26, 27) in einem oberen Auflagebereich der Fördereinrichtung (2) vorgesehen ist, wobei die Scaneinheit (6) während des Scannens gemeinsam mit der Bandlücke (16, 26, 27) der Fördereinrichtung (2) verfahren wird, und das Scannen innerhalb der Bandlücke (16, 26, 27) erfolgt.

2. Scanvorrichtung nach Anspruch 1, weiterhin umfassend ein Schutzgehäuse (13), mit einem Innenraum, der dazu ausgebildet ist, dass der wenigstens eine Lebensmittelriegel (1, 28, 29, 30) während des Scanvorgangs in diesem angeordnet ist, wobei die Länge des Innenraums des Schutzgehäuses (13) in Längsrichtung (L) kürzer ist als die zweifache Länge eines Lebensmittelriegels (1, 28, 29, 30).

3. Scanvorrichtung nach einem der vorangehenden Ansprüche, wobei zwischen dem oberen Auflagebereich und einem unteren Rücklaufbereich des Fördermittels (3) eine Detektoreinheit (9) der Scaneinheit (6) vorgesehen ist.

4. Scanvorrichtung nach Anspruch 1, wobei die Fördereinrichtung (2) als zwei Rückzugsbänder ausgestaltete Fördermittel (20, 21) aufweist, deren in Längsrichtung (L) verfahrbare Umlenkrollen (17, 18) die Ränder der Bandlücke (16, 26, 27) definieren.

5. Scanvorrichtung nach einem der vorangehenden Ansprüche, wobei die Umlenkrollen (17, 18) mechanisch in Längsrichtung (L) gekoppelt sind, um eine gleichbleibende Länge der Bandlücke (16, 26, 27) zu gewährleisten, und wobei diese Kopplung insbesondere auch die Scaneinheit (6) umfasst.

6. Scanvorrichtung nach einem der vorangehenden Ansprüche, wobei die Fördereinrichtung (2) mehrere parallele Förderspuren aufweist.

7. Scanvorrichtung nach Anspruch 6, wobei die Scaneinheit zwischen den Förderspuren in Querrichtung (Q) verfahrbar ist.

8. Scanvorrichtung nach Anspruch 6 oder 7, wobei jeweils eine in Längsrichtung (L) verfahrbare Scaneinheit pro Förderspur oder pro einer Untergruppe von Förderspuren vorgesehen ist.

9. Verfahren zum Scannen von Lebensmittelriegeln (1, 28, 29, 30), mit den folgenden Schritten:
Fördern eines Lebensmittelriegels (1, 28, 29, 30) in einer Längsrichtung (L) mittels einer Fördereinrichtung (2), und
Scannen des Lebensmittelriegels (1, 28, 29, 30) mittels einer Scaneinheit (6),
Verfahren der Scaneinheit (6) entlang des Lebensmittelriegels (1, 28, 29, 30) in Längsrichtung (L) während des Scannens,
**dadurch gekennzeichnet, dass**
die Scaneinheit (6) während des Scannens gemeinsam mit einer Bandlücke (16, 26, 27) der Fördereinrichtung (2) verfahren wird, und das Scannen innerhalb der Bandlücke (16, 26, 27) erfolgt, wobei die äußere und/oder innere Gestalt des Lebensmittelriegels (1, 28, 29, 30) erfasst wird.

10. Verfahren nach Anspruch 9, wobei das Fördern des Lebensmittelriegels (1, 28, 29, 30) das Einfahren des Lebensmittelriegels (1, 28, 29, 30) in ein Schutzgehäuse (13) umfasst, und die Scaneinheit (6) nur innerhalb des Schutzgehäuses (13) verfahren wird.

11. Verfahren nach einem der Ansprüche 9 oder 10, wobei Lebensmittelriegel (1, 28, 29, 30) in mehreren parallellen Spuren gefördert werden, und wobei die Scaneinheit (6) zwischen den Scanvorgängen in Querrichtung (Q) zwischen den Spuren verfahren wird.

12. Verfahren nach einem der Ansprüche 9 bis 11, wobei die Scaneinheit (6) bei aufeinanderfolgenden Scanvorgängen jeweils in entgegengesetzte Richtungen verfahren wird.

13. Verfahren nach einem der Ansprüche 9 bis 12, wobei der Lebensmittelriegel (1, 28, 29, 30) beim Scannen durchstrahlt wird, und das Ergebnis des Scanvorgangs mit Referenzdaten korrigiert wird, die bei einem Scanvorgang ohne Lebensmittelriegel (1, 28, 29, 30) ermittelt wurden.

## Claims

1. A scanning device for scanning food bars (1, 28, 29, 30) for a food processing system, comprising:
a conveyor (2) for conveying at least one food bar (1, 28, 29, 30) in a longitudinal direction (L), and
a scanning unit (6) adapted to detect the outer and/or inner shape of the at least one food bar (1, 28, 29, 30), wherein
the scanning unit (6) is movable at least along the longitudinal direction (L), wherein the conveyor (2) comprises a circulating conveyor means (3, 20, 21), and
**characterized in that**
a belt gap (16, 26, 27) which is delimited by return pulleys (17, 18) and can be moved in the longitudinal direction (L) is provided in an upper support region of the conveyor (2), wherein the scanning unit (6) is moved together with the belt gap (16, 26, 27) of the conveyor (2) during scanning and the scanning takes place within the belt gap (16, 26, 27).

2. The scanning device according to claim 1, further comprising a protective housing (13) having an interior space configured to have the at least one food bar (1, 28, 29, 30) disposed therein during the scanning process, wherein the length of the interior space of the protective housing (13) in the longitudinal direction (L) is shorter than twice the length of a food bar (1, 28, 29, 30).

3. The scanning device according to any one of the preceding claims, wherein a detector unit (9) of the scanning unit (6) is provided between the upper support region and a lower return side of the conveying means (3).

4. The scanning device according to claim 1, wherein the conveying device (2) includes conveying means (20, 21) designed as two return belts, the return pulleys (17, 18) of which that can be moved in the longitudinal direction (L) define the edges of the belt gap (16, 26, 27).

5. The scanning device according to any one of the preceding claims, wherein the return pulleys (17, 18) are mechanically coupled in the longitudinal direction (L) to ensure a constant length of the band gap (16, 26, 27), and wherein this coupling in particular also includes the scanning unit (6).

6. The scanning device according to any one of the preceding claims, wherein the conveying device (2) includes a plurality of parallel conveying tracks.

7. The scanning device according to claim 6, wherein the scanning unit is movable in the transverse direction (Q) between the conveying tracks.

8. The scanning device according to claims 6 or 7, wherein one scanning unit movable in the longitudinal direction (L) is provided per conveyor track or per one subgroup of conveyor tracks.

9. A method for scanning food bars (1, 28, 29, 30), comprising the following steps:
conveying a food bar (1, 28, 29, 30) in a longitudinal direction (L) by means of a conveyor (2), and scanning the food bar (1,28, 29, 30) by means of a scanning unit (6),
moving the scanning unit (6) along the food bar (1, 28, 29, 30) in the longitudinal direction (L) during scanning,
**characterized in that**
the scanning unit (6) is moved together with a belt gap (16, 26, 27) of the conveyor (2) during scanning, and the scanning takes place within the belt gap (16, 26, 27) wherein the outer and/or inner shape of the food bar (1, 28, 29, 30) are/is detected.

10. The method according to claim 9, wherein conveying the food bar (1, 28, 29, 30) comprises moving the food bar (1, 28, 29, 30) into a protective housing (13), and the scanning unit (6) is moved only within the protective housing (13).

11. The method according to any one of claims 9 or 10, wherein food bars (1, 28, 29, 30) are conveyed in a plurality of parallel tracks, and wherein between the scanning operations the scanning unit (6) is moved between the tracks in the transverse direction (Q).

12. The method according to any one of claims 9 to 11, wherein the scanning unit (6) is moved in respective opposite directions during successive scanning operations.

13. The method according to any one of claims 9 to 12, wherein the food bar (1, 28, 29, 30) is transilluminated during scanning, and the result of the scanning process is corrected by means of reference data determined during a scanning process without food bars (1, 28, 29, 30).

## Revendications

1. Dispositif de balayage permettant de scanner des barres alimentaires (1, 28, 29, 30) pour un système de traitement des aliments, comprenant
un dispositif de transport (2) permettant de transporter au moins une barre alimentaire (1, 28, 29, 30) dans une direction longitudinale (L), et
une unité de balayage (6) conçue pour détecter la forme extérieure et/ou intérieure d'au moins une barre alimentaire (1, 28, 29, 30), dans lequel
l'unité de balayage (6) peut être déplacée au moins le long de la direction longitudinale (L), dans lequel le dispositif de transport (2) comprend un moyen de transport (3, 20, 21) périphérique, et
**caractérisé en ce que**
une fente de bande (16, 26, 27) limitée par des rouleaux de renvoi (17, 18) et pouvant être déplacée dans le sens longitudinal (L) est fournie dans une région d'appui supérieure du dispositif de transport (2), dans lequel l'unité de balayage (6) est déplacée en commun avec la fente de bande (16, 26, 27) du dispositif de transport (2) pendant le balayage, et le balayage a lieu à l'intérieur de la fente de bande (16, 26, 27).

2. Dispositif de balayage selon la revendication 1, comprenant en outre un boîtier de protection (13), avec un espace intérieur qui est conçu pour que la au moins une barre alimentaire (1, 28, 29, 30) soit agencée dans celui-ci pendant l'opération de balayage, dans lequel la longueur de l'espace intérieur du boîtier de protection (13) dans la direction longitudinale (L) est plus courte que deux fois la longueur d'une barre alimentaire (1, 28, 29, 30).

3. Dispositif de balayage selon l'une quelconque des revendications précédentes, dans lequel une unité de détection (9) de l'unité de balayage (6) est fournie entre la région d'appui supérieure et une région de retour inférieure du moyen de transport (3).

4. Dispositif de balayage selon la revendication 1, dans lequel le dispositif de transport (2) présente des moyens de transport (20, 21) conçus sous la forme de deux sangles de retrait, dont les rouleaux de renvoi (17, 18) pouvant être déplacés dans la direction longitudinale (L) définissent les bords de la fente de bande (16, 26, 27).

5. Dispositif de balayage selon l'une quelconque des revendications précédentes, dans lequel les rouleaux de renvoi (17, 18) sont couplés de manière mécanique dans la direction longitudinale (L) afin de garantir une longueur constante de la fente de bande (16, 26, 27), et dans lequel ledit couplage comprend en particulier également l'unité de balayage (6).

6. Dispositif de balayage selon l'une quelconque des revendications précédentes, dans lequel le dispositif de transport (2) présente plusieurs voies de transport parallèles.

7. Dispositif de balayage selon la revendication 6, dans lequel l'unité de balayage peut être déplacée entre les voies de transport dans la direction transversale (Q).

8. Dispositif de balayage selon la revendication 6 ou 7, dans lequel une unité de balayage pouvant être déplacée dans la direction longitudinale (L) est fournie pour chaque voie de transport ou pour chaque sous-groupe de voies de transport.

9. Procédé de balayage de barres alimentaires (1, 28, 29, 30), comprenant les étapes ci-dessous consistant à :
transporter une barre alimentaire (1, 28, 29, 30) dans une direction longitudinale (L) au moyen d'un dispositif de transport (2), et
scanner la barre alimentaire (1, 28, 29, 30) au moyen d'une unité de balayage (6),
déplacer l'unité de balayage (6) le long de la barre alimentaire (1, 28, 29, 30) dans le sens longitudinal (L) pendant le balayage,
**caractérisé en ce que**
l'unité de balayage (6) est déplacée en commun avec une fente de bande (16, 26, 27) du dispositif de transport (2) pendant le balayage, et le balayage intervient à l'intérieur de la fente de bande (16, 26, 27), dans lequel la forme extérieure et/ou intérieure de la barre alimentaire (1, 28, 29, 30) est détectée.

10. Procédé selon la revendication 9, dans lequel le transport de la barre alimentaire (1, 28, 29, 30) comprend l'étape consistant à introduire la barre alimentaire (1, 28, 29, 30) dans un boîtier de protection (13), et l'unité de balayage (6) est déplacée uniquement à l'intérieur du boîtier de protection (13).

11. Procédé selon l'une quelconque des revendications 9 ou 10, dans lequel des barres alimentaires (1, 28, 29, 30) sont transportées sur plusieurs voies parallèles, et dans lequel l'unité de balayage (6) est déplacée entre les voies dans la direction transversale (Q) entre les opérations de balayage.

12. Procédé selon l'une quelconque des revendications 9 à 11, dans lequel l'unité de balayage (6) est déplacée dans des directions opposées lors d'opérations de balayage successives.

13. Procédé selon l'une quelconque des revendications 9 à 12, dans lequel la barre alimentaire (1, 28, 29, 30) est traversée par un rayonnement lors du balayage, et le résultat de l'opération de balayage est corrigé avec des données de référence qui ont été déterminées lors d'une opération de balayage sans barre alimentaire (1, 28, 29, 30).
